# EUROPEAN PATENT APPLICATION

(11) **EP 4 789 695 A1**
(43) Date of publication of application: **12.08.2026**
(21) Application number: 25857977.0
(22) Date of filing: 15.12.2025
(51) Int. Cl.: A61M 1/00, A61M 25/00

(54) **DRAINAGE TUBE AND DRAINAGE APPARATUS**

(30) Priority: 20.12.2024 CN 202423168433 U; 29.07.2025 CN 202511050181
(71) Applicant: Micro-Tech (Nanjing) Co., Ltd., Nanjing, Jiangsu 210032 (CN); Sena, Flaubert, Nanjing, Jiangsu 210032 (CN)
(72) Inventor: SENA, Flaubert, Nanjing, Jiangsu 210032 (CN); LIN, Qi, Nanjing, Jiangsu 210032 (CN); HU, Jie, Nanjing, Jiangsu 210032 (CN); JIN, Hongyan, Nanjing, Jiangsu 210032 (CN); TANG, Zhi, Nanjing, Jiangsu 210032 (CN); BRUGNEROTTO, Ricardo, Nanjing, Jiangsu 210032 (CN)
(74) Representative: Lapienis, Juozas
(86) International application number: PCT/CN2025/142612
(87) International publication number: WO 2026/130303

(57) **Abstract**

An embodiment of the present application provides a drainage tube and a drainage device, which relates to the technical field of medical devices. The drainage tube includes a drainage portion, a porous loose structure and a granulation barrier structure. The drainage portion has a first drainage channel, and the drainage portion is provided with a first drainage opening in communication with the first drainage channel. The drainage portion is covered with the porous loose structure. The porous loose structure is covered with the granulation barrier structure. The granulation barrier structure has a second drainage opening, wherein the second drainage opening, the porous loose structure, the first drainage opening and the first drainage channel are in communication. With the provision of a granulation barrier structure, while the requirements of negative pressure adsorption can be met, the granulation is prevented from growing into the porous loose structure, thereby reducing the bleeding of granulation tissue and improving the therapeutic effect of negative pressure drainage.

## Description

### CROSS-REFERENCE TO THE ERELATED APPLICATIONS

The present application claims priority to Chinese Patent Application No. 202423168433.6, filed with the China National Intellectual Property Administration on December 20, 2024, and entitled "DRAINAGE TUBE AND DRAINAGE DEVICE"; and priority to Chinese Patent Application No. 202511050181.3, filed with the China National Intellectual Property Administration on July 29, 2025, and entitled "DRAINAGE TUBE AND DRAINAGE DEVICE", the contents of which are incorporated herein by reference in entirety.

### TECHNICAL FIELD

The present application relates to the technical field of medical devices, specifically relates to a drainage tube and a drainage device.

### BACKGROUND

In recent years, endoscopic vacuum therapy (EVT) has become a safe and effective method for treating gastrointestinal perforation, fistulas, and anastomotic leakage. In this method, a sponge is arranged at a distal end of a drainage tube and inserted into the fistula. After a proximal end of the drainage tube is connected to a negative pressure instrument, negative pressure drainage therapy is performed on the fistula. Through continuous negative pressure suction, liquids such as the exudate from the wound or gastrointestinal secretions are continuously and effectively aspirated, promoting perfusion and granulation tissue hyperplasia.

However, during the negative pressure drainage therapy, granulation will grow into the interior of the sponge, causing the granulation tissue and the sponge to be pulled against each other during the subsequent removal of the drainage tube, resulting in bleeding and affecting the therapeutic effect.

### SUMMARY

The objective of the present application is to provide a drainage tube and drainage device, which can reduce the bleeding of granulation tissue and improve the therapeutic effect of negative pressure drainage.

The embodiments of the present application are implemented as follows.

In the first aspect, the present application provides a drainage tube including:
a drainage portion, wherein the drainage portion has a first drainage channel, and the drainage portion is provided with a first drainage opening in communication with the first drainage channel;
a porous loose structure, wherein the drainage portion is covered with the porous loose structure; and
a granulation barrier structure, wherein the porous loose structure is covered with the granulation barrier structure, and the granulation barrier structure is provided with a second drainage opening,
wherein the second drainage opening, the porous loose structure, the first drainage opening and the first drainage channel are in communication.

In an optional embodiment, a plurality of first drainage openings are provided, and each of the plurality of first drainage openings is in communication with the first drainage channel and the porous loose structure, and wherein a plurality of the second drainage openings are provided, and each of the plurality of second drainage openings is communicated with the porous loose structure.

In an optional embodiment, the plurality of first drainage openings are arranged at intervals along an extension direction of the drainage portion; and/or
the plurality of second drainage openings are arranged at intervals along an extension direction of the granulation barrier structure.

In an optional embodiment, the second drainage opening is in number which is greater than or equal to that of the first drainage opening, and the diameter of the second drainage opening is smaller than the diameter of the first drainage opening.

In an optional embodiment, the drainage tube further comprises a drainage tube body, wherein a distal end of the drainage tube body is connected to a proximal end of the drainage portion, and the drainage tube body has a second drainage channel in communication with the first drainage channel.

In an optional embodiment, a distal end of the drainage portion is provided with a first opening in communication with the first drainage channel, and a proximal end of the drainage tube body is provided with a second opening in communication with the second drainage channel.

In an optional embodiment, a hardness of the proximal end of the drainage tube body is greater than or equal to a hardness of the drainage portion.

In the second aspect, a drainage device provided by the present application includes a drainage tube and a negative pressure device, and the negative pressure device is connected to a proximal end of the drainage portion and is in communication with the first drainage channel.

In an optional embodiment, the drainage device further comprises a connector, wherein the connector is detachably arranged at the proximal end of the drainage portion, and the negative pressure device is connected to a proximal end of the connector.

In an optional embodiment, the drainage tube further comprises a first support pipe and a connection end cap, the drainage tube further comprises a first support pipe and a connection end cap, the first support pipe is disposed as passing through an interior of the drainage tube body and the interior of the drainage portion, and the first support pipe is configured to support the inner walls of the drainage portion and the drainage tube body, wherein the connection end cap is connected to a proximal end of the first support pipe, and a central protrusion of the connection end cap forms an insertion portion that can be inserted into the drainage tube body.

In an optional embodiment, the first support pipe is configured as a solid structure, or, the first support pipe is configured as a hollow structure to form a hollow space, and the connection end cap has a through hole communicated with the hollow space to form a communication channel for a guidewire to pass through.

In an optional embodiment, the drainage device further comprises a second support pipe;
wherein the second support pipe includes a straight barrel section and an overlap section connected with each other; and
wherein the straight barrel section is configured to be arranged inside a proximal end of the drainage portion, and the overlap section is configured to abut against a proximal end opening of the drainage portion.

The beneficial effects of the embodiments of the present application include the following:
The drainage tube comprises a drainage portion, a porous loose structure and a granulation barrier structure. The drainage portion has a first drainage channel, and the drainage portion is provided with a first drainage opening, which is in communication with the first drainage channel. The drainage portion is covered with the porous loose structure, and the porous loose structure is covered with the granulation barrier structure, and the granulation barrier structure is provided with a second drainage opening, wherein the second drainage opening, the porous loose structure, the first drainage opening and the first drainage channel are in communication.

It is easy to understand that, the porous loose structure serves to communicate the second drainage opening with the first drainage opening. During negative pressure drainage therapy, liquids such as the exudate from the wound at the fistula can pass through the second drainage opening, the porous loose structure and the first drainage opening, and enter the first drainage channel of the drainage portion. Then, under the action of negative pressure, the liquid is drawn out of the drainage tube. Moreover, as the porous loose structure is covered with the granulation barrier structure, the granulation barrier structure can, to a certain extent, prevent granulation tissue from growing into the porous loose structure. Thus, during the subsequent removal of the drainage tube, the pulling between the granulation tissue and the granulation barrier structure as well as the porous loose structure can be avoided, thereby reducing the bleeding of granulation tissue and improving the therapeutic effect of negative pressure drainage.

The drainage device includes the drainage tube, and the drainage device has all the beneficial effects of the drainage tube.

### BRIEF DESCRIPTION OF THE DRAWINGS

To more clearly illustrate the technical solutions of the embodiments of the present application, a brief introduction to the drawings used in the embodiments will be provided below. It should be understood that the following drawings only show some embodiments of the present application and should not be construed as a limitation of the scope. For those skilled in the art, other relevant drawings can also be obtained based on these drawings without inventive efforts.
FIG. 1 is a use schematic diagram of a drainage device provided by the embodiment of the present application;
FIG. 2 is a schematic structural diagram of a drainage tube provided by the embodiment of the present application;
FIG. 3 is an explosive view of the drainage tube provided by the embodiment of the present application;
FIG. 4 is a sectional view of the drainage tube provided by the embodiment of the present application;
FIG. 5 is a sectional view of a drainage tube with a first support pipe provided in the embodiment of the present application;
FIG. 6 is a perspective schematic structural diagram of a drainage tube with a connection end cap provided in the embodiment of the present application;
FIG. 7 is a front view schematic structural diagram of a drainage tube with a connection end cap provided in the embodiment of the present application;
FIG. 8 is a sectional structural view of a drainage tube with a connection end cap and a first support pipe provided in the embodiment of the present application;
FIG. 9 is a sectional structural view of the drainage tube with a connection end cap and a second support pipe provided in the embodiment of the present application;
FIG. 10 shows a schematic structural diagram of two different embodiments of the second support pipe; and
FIG. 11 is a use schematic diagram of a drainage tube provided in the embodiment of the present application.

Reference signs: 1000 - drainage device; 100 - drainage tube; 10 - drainage portion; 11 - first drainage channel; 12 - first drainage opening; 13 - first opening; 20 - porous loose structure; 30 - granulation barrier structure; 31 - second drainage opening; 40 - drainage tube body; 41 - second drainage channel; 42 - second opening; 50 - first support pipe; 60 - connection end cap; 61 - insertion portion; 62 - slide groove; 70 - second support pipe; 71 - straight barrel section; 72 - overlap section; 200 - negative pressure device; 300 - connector; 310 - male connector; 311 - lug; 320 - female connector; and 330 - sealing ring.

### DETAILED DESCRIPTION OF THE EMBODIMENTS

To make the objective, technical solutions and advantages of the embodiments of the present application clearer, the technical solutions of the embodiments of the present application will be described clearly and completely in combination with the drawings in the embodiments of the present application. Obviously, the described embodiments are only a part but not all of the embodiments of the present application. The assemblies of the embodiments of the present application generally described and shown in the drawings here can be arranged and designed in various different configurations.

Therefore, the following detailed description of the embodiments of the present application provided in the drawings is not intended to limit the scope of protection of the present application as claimed, but merely represents selected embodiments of the present application. All other embodiments obtained by an ordinary person skilled in the art without inventive efforts based on the embodiments of the present application fall within the scope of protection of the present application.

It should be noted that similar reference numerals and letters in the drawings below represent similar items. Therefore, once an item is defined in one drawing, it does not need to be further defined or explained in subsequent drawings.

In the description of the present application, it should be noted that the orientation or positional relationship indicated by the terms "center", "up", "down", "left", "right", "vertical", "horizontal", "inside", "outside", etc. is based on the orientation or positional relationship shown in the drawings, or the orientation or positional relationship that is commonly placed when the product of the invention is in use. It is only for the convenience of describing the present application and simplifying the description, and does not indicate or imply that the device or element referred to must have a specific orientation, be constructed and operated in a specific orientation, and therefore should not be construed as a limitation of the present application. Furthermore, terms such as "first", "second", and "third" are only used to distinguish descriptions and should not be understood as indicating or implying relative importance.

Furthermore, terms such as "horizontal" and "vertical" do not imply that the components must be absolutely horizontal or vertically suspended, but rather can be slightly inclined. For instance, "horizontal" merely means that the direction is more horizontal compared to "vertical", and it does not imply that the structure must be completely horizontal; rather, it can be slightly inclined.

In the description of the present application, it should also be noted that, unless otherwise explicitly specified and limited, the terms "configure", "mount", "connect" and "link" should be understood in a broad sense. For example, it can be a fixed connection, a detachable connection, or an integrated connection. It can be a mechanical connection or an electrical connection. It can be a direct connection, an indirect connection through an intermediate medium, or an internal communication between two elements. For those skilled in the art, the specific meanings of the above terms in the present application can be understood in light of the specific circumstances.

As described in the part "BACKGROUND", endoscopic vacuum therapy (EVT) is a method for treating gastrointestinal perforation, fistulas, and anastomotic leakage. In this method, a sponge is arranged at a distal end of a drainage tube and inserted into the fistula. After a proximal end of the drainage tube is connected to a negative pressure instrument, negative pressure drainage therapy is performed on the fistula. However, during the negative pressure drainage therapy, granulation will grow into the interior of the sponge, causing the granulation tissue and the sponge to be pulled against each other during the subsequent removal of the drainage tube, resulting in bleeding and affecting the therapeutic effect.

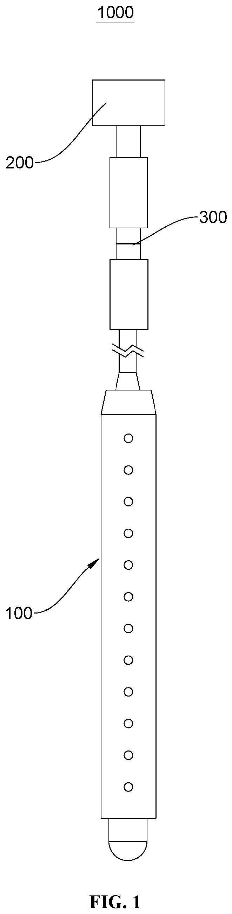

Based on this, with reference to FIGS. 1 to 4, the embodiment of the present application provides a drainage tube 100 and a drainage device 1000, which can effectively improve the technical problems mentioned above, that is, they can reduce the bleeding of granulation tissue and improve the therapeutic effect of negative pressure drainage. The following will provide a detailed description of the drainage tube 100 and the drainage device 1000.

With reference to FIG. 1, FIG. 1 is a schematic structural diagram of the drainage device 1000 provided in the embodiment. As shown in FIG. 1, the drainage device 1000 includes a drainage tube 100 and a negative pressure device 200. The negative pressure device 200 is connected to the proximal end of the drainage tube 100. That is, during negative pressure drainage therapy, the distal end of the drainage tube 100 is inserted into the fistula. Under the negative pressure provided by the negative pressure device 200, liquids such as the wound exudate, etc. at the fistula will be drawn into an internal channel of the drainage tube 100 and then discharged from the proximal end of the drainage tube 100, thereby achieving the removal of exudate, promoting wound healing, reducing the risk of infection and accelerating tissue repair.

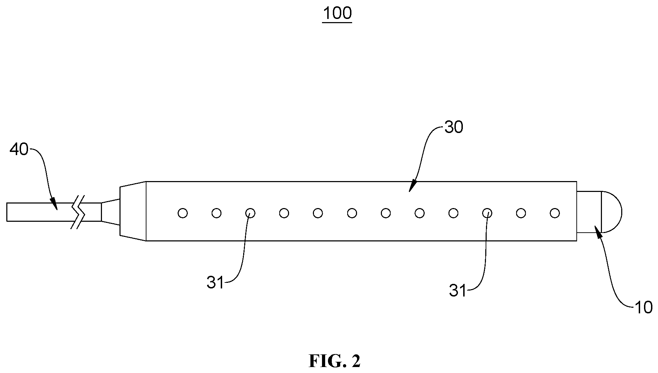

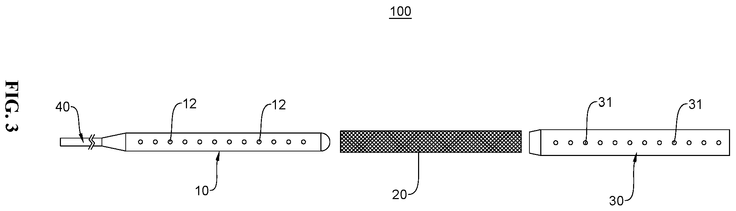

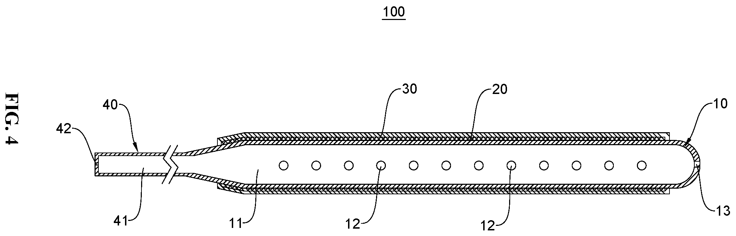

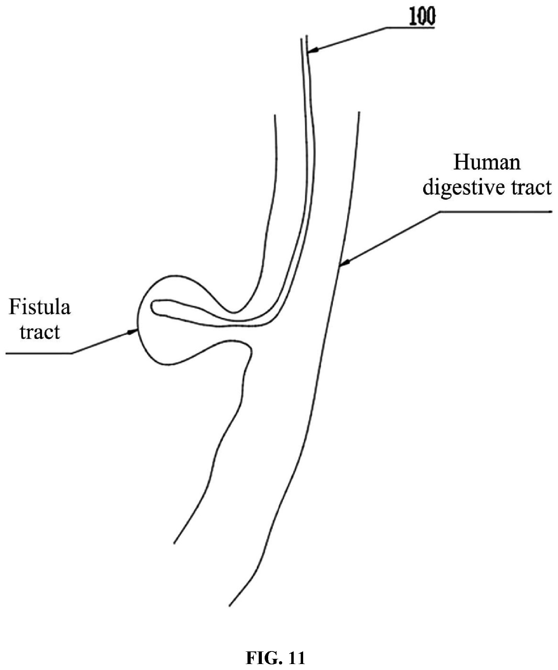

Furthermore, please refer to FIGS. 2 to 4 and FIG. 11. FIG. 2 is a schematic structural diagram of the drainage tube 100 provided in the embodiment. FIG. 3 is an explosive view of the drainage tube 100 provided in the embodiment. FIG. 4 is a sectional view of the drainage tube 100 provided in the embodiment. FIG. 11 is a schematic diagram of the drainage tube 100 provided in the embodiment being placed into a fistula tract in the human tissue. Referring to FIGS. 2 to 4, the drainage tube 100 includes a drainage portion 10, a porous loose structure 20 and a granulation barrier structure 30. The drainage portion 10 has a first drainage channel 11, and the drainage portion 10 is provided with a first drainage opening 12. The first drainage opening 12 is in communication with the first drainage channel 11. The drainage portion 10 is covered with the porous loose structure 20, and the porous loose structure 20 is covered with the granulation barrier structure 30. The granulation barrier structure 30 is provided with a second drainage opening 31, where the second drainage opening 31, the porous loose structure 20, the first drainage opening 12 and the first drainage channel 11 are in communication.

Referring to FIGS. 1 to 4, in the embodiment, the negative pressure device 200 is connected to the proximal end of the drainage portion 10 and is in communication with the first drainage channel 11. It is easy to understand that the porous loose structure 20 serves to communicate the second drainage opening 31 with the first drainage opening 12. During negative pressure drainage therapy, liquids such as the wound exudate, etc. at the fistula can pass through the second drainage opening 31, the porous loose structure 20 and the first drainage opening 12, and enter the first drainage channel 11 of the drainage portion 10. Then, under the effect of negative pressure, the liquid is drawn out of the drainage tube 100. Moreover, as the porous loose structure 20 is covered with the granulation barrier structure 30, the granulation barrier structure 30 can, to a certain extent, prevent granulation from growing into the porous loose structure 20. Thus, during the subsequent removal operation of the drainage tube 100, the pulling between the granulation tissue and the granulation barrier structure 30 as well as the porous loose structure 20 can be avoided, thereby reducing the bleeding of the granulation tissue, and improving the therapeutic effect of negative pressure drainage.

For the convenience of inserting the drainage tube 100 entirely into the body and connecting the drainage tube 100 with the negative pressure device 200 after the drainage tube 100 extending out of the body, in the embodiment, the drainage tube 100 further includes a drainage tube body 40. The distal end of the drainage tube body 40 is connected to the proximal end of the drainage portion 10, and the drainage tube body 40 has a second drainage channel 41. The second drainage channel 41 is in communication with the first drainage channel 11. That is, the proximal end of the drainage tube body 40 is connected to the negative pressure device 200, and the second drainage channel 41 is communicated with the negative pressure device 200. Under the negative pressure of the negative pressure device 200, liquids such as the exudate from the wound, etc. pass through the first drainage channel 11 and the second drainage channel 41 in sequence and are discharged from the proximal end of the drainage tube body 40.

In addition, the hardness of the proximal end of the drainage tube body is greater than or equal to the hardness of the drainage portion. The proximal end of the drainage tube body has a higher hardness, which enables the drainage tube body to have good support and pushing force during puncture or placement, facilitating the physician to precisely control the path of the drainage tube entering the body and reducing the difficulty of operation. The hardness of the drainage portion is relatively low, which brings good flexibility and compliance, so that the drainage portion can adapt to the complex anatomical structure of the internal cavity or organ, reduce the stimulation and damage to the surrounding tissues. It should be noted that the drainage tube and the drainage portion are integrally formed as one piece. During the production process, different hardness agents are added to achieve different hardness levels at the proximal end of the drainage tube and the drainage portion, which are situated at the two sides of the structure.

With reference to FIGS. 1 and 2, to facilitate the detachment and mounting of the drainage tube 100 and the negative pressure device 200, the drainage device 1000 further includes a connector 300. The connector 300 is detachably arranged at the proximal end of the drainage portion 10. Specifically, in the embodiment, the fixing connector 300 is detachably arranged at the proximal end of the drainage tube body 40. The negative pressure device 200 is connected to the proximal end of the connector 300.

By configuring the connector 300 in a detachable way, it is convenient for the operation of placing the drainage tube 100. That is, the drainage tube 100 is inserted into the human body first, then the connector 300 is removed. After the proximal end of the drainage tube 100 extends out of the body, the connector 300 is reconnected. In this way, during the operation of placing the drainage tube 100, the connector 300 can be prevented from interfering with the tube walls of organs such as the digestive tract, intestines, nasal cavity, etc. which may cause damage. At the same time, the smooth placement and removal of the drainage tube 100 can also be ensured. To facilitate the placement of the drainage tube 100 into the body, in the embodiment, the drainage device 1000 further includes a guidewire (not shown). The guidewire is configured to extend through the drainage tube 100 and has a guiding function, enabling the drainage tube 100 to be accurately delivered to the placement site.

Specifically, referring to FIG. 4, the distal end of the drainage portion 10 is provided with a first opening 13 in communication with the first drainage channel 11. The proximal end of the drainage tube body 40 is provided with a second opening 42 in communication with the second drainage channel 41. In this way, when the drainage tube 100 is delivered to the placement site by means of the guidewire, the guidewire passes through the first opening 13, the first drainage channel 11, the second drainage channel 41 and the second opening 42 in sequence. After the drainage tube 100 is delivered as a whole to the target position, the guidewire is removed from the second opening 42.

Furthermore, in order to enhance the liquid suction effect by the negative pressure, a plurality of the first drainage openings 12 can be provided, and each of the plurality of first drainage openings 12 is communicated with the first drainage channel 11 and the porous loose structure 20. A plurality of the second drainage openings 31 can also be provided, and each of the plurality of second drainage openings 31 is communicated with the porous loose structure 20.

With reference to FIGS. 3 and 4, it should be noted that in the embodiment, a plurality of first drainage openings 12 are arranged at intervals along an extension direction of the drainage portion 10, which can transfer the liquid drawn by the porous loose structure 20 into the internal channel of the drainage portion 10, that is, into the first drainage channel 11, at multiple different positions, thereby improving the liquid suction effect by the negative pressure, which further enhances the therapeutic effect.

Of course, in other embodiments, the arrangement of the plurality of first drainage openings 12 is not limited to this. A plurality of first drainage openings 12 can also be arranged at intervals in a circular way, or in a staggered way, or in a spiral way or in an array, etc.

With reference to FIGS. 2 and 3, in order to enhance the liquid suction effect by the negative pressure, in the embodiment, a plurality of second drainage openings 31 are arranged at intervals along an extension direction of the granulation barrier structure 30, which can transfer liquids such as the wound exudate , etc. into the porous loose structure 20 from multiple different positions, thereby achieving liquid suction at different parts of the wound of the fistula, improving the therapeutic effect of negative pressure drainage.

Similarly, in other embodiments, the arrangement of the plurality of second drainage openings 31 is not limited to this. A plurality of second drainage openings 31 can also be arranged at intervals in a circular way, or in a staggered way, or in a spiral way or in an array, etc.

Furthermore, in the embodiment, the number of the second drainage openings 31 is greater than or equal to the number of the first drainage openings 12, thereby making the second drainage openings denser than the first drainage openings, and the diameter of the second drainage opening 31 is smaller than the diameter of the first drainage opening 12, which can achieve a more uniform liquid distribution in the local area, reduce the risk of uneven impact force or tissue damage caused by excessive local flow velocity, and is suitable for clinical scenarios with high requirements for gentleness during the drainage process. In addition, configuring a finer drainage structure at the tip area in contact with the tissue can reduce the degree of stimulation to the surrounding tissues, improve the adaptability and comfort of the device in the body, and is especially suitable for long-term indwelling medical devices.

In addition, as an alternative technical solution, a plurality of first drainage openings 12 can also be arranged in a one-to-one correspondence with a plurality of second drainage openings 31. This can shorten the flow path through which liquids such as the exudate, etc. enter the first drainage channel 11 of the drainage portion 10, thereby further enhancing the suction effect by negative pressure and correspondingly improving the therapeutic effect of negative pressure drainage.

Furthermore, referring to FIG. 3, the size of the drainage portion 10 is larger than the size of the drainage tube body 40. It should be noted that in case both the drainage portion 10 and the drainage tube body 40 are cylindrical in shape, the size relationship mentioned above can be specifically understood as the diameter of the drainage portion 10 being larger than the diameter of the drainage tube body 40. That is, the drainage tube 100 as a whole can be regarded as a tube with variable diameters. The section of the drainage tube arranged at the wound site of the fistula has a larger diameter. Referring to FIGS. 3 and 4, it can be understood that the size of the first drainage channel 11 is larger than the size of the second drainage channel 41. With such configuration, the area of the first drainage channel 11 through which liquids such as the wound exudate, etc. flow can be increased, which can ensure the flow of a larger amount of liquid and improve the suction effect by subsequent negative pressure. Moreover, the configuration where the drainage tube body 40 being relatively small can facilitate the passage of the drainage tube body 40 through the internal channels of organs such as the digestive tract, intestines, and nasal cavity.

It should be noted that in the embodiment, the porous loose structure 20 is specifically gauze with properties such as good air permeability, water absorption and softness, and can ensure the therapeutic effect of negative pressure drainage. Moreover, gauze also has a certain filtering function, so that liquids such as exudate, etc. can be filtered to a certain extent. With liquids absorbed to ensure the dryness of the wound, the gauze can also serve to clean, thereby reducing the risk of wound infection.

Of course, in other embodiments, the porous loose structure 20 can also be a sponge, which also has the properties of air permeability, water absorption, softness and filterability, etc., and can serve to absorb water, filter, dry and clean the wound. It is easy to understand that the specific material of the porous loose structure 20 is not limited to this, as long as the material has the properties of being porous and loose, can enable the passage of liquid, ensuring that the liquid flowing in from the second drainage opening 31 can smoothly pass through and enter the first drainage opening 12.

Additionally, it should be noted that in the embodiment, the granulation barrier structure 30 is in particular a heat shrink tube, which has the properties of sealing and waterproofing, corrosion resistance, flexibility and wear resistance, etc., can effectively prevent granulation tissue from growing into the porous loose structure 20, and at the same time avoid the leakage of liquid within the porous loose structure 20.

Of course, in other embodiments, the granulation barrier structure 30 can also be a protective film or other structures with protective functions, as long as the granulation barrier structure 30 can ensure that the granulation tissue is blocked from growing into the porous loose structure 20 and the leakage of liquid from the porous loose structure 20 is avoided.

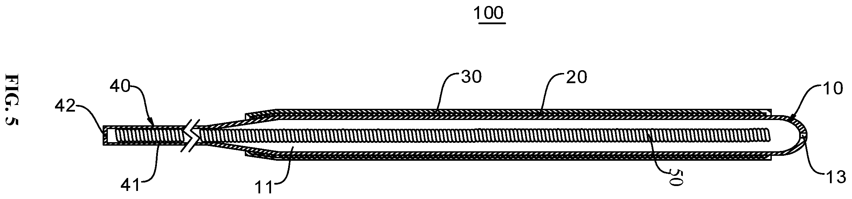

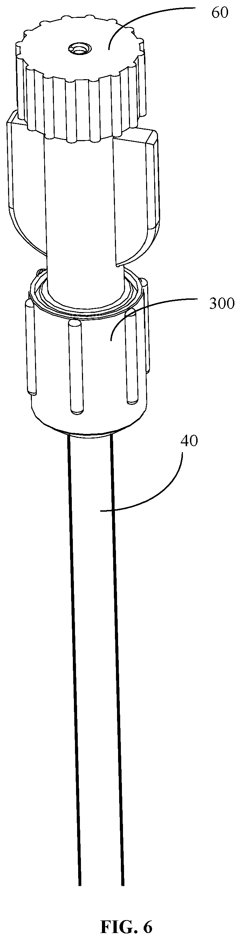

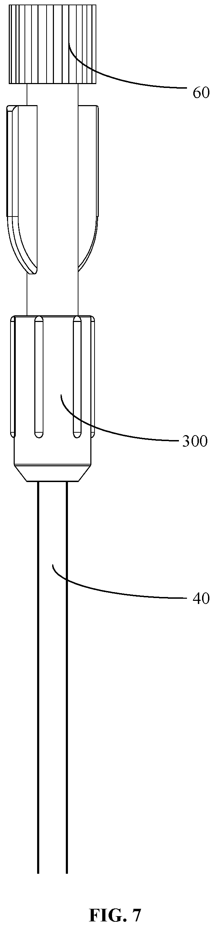

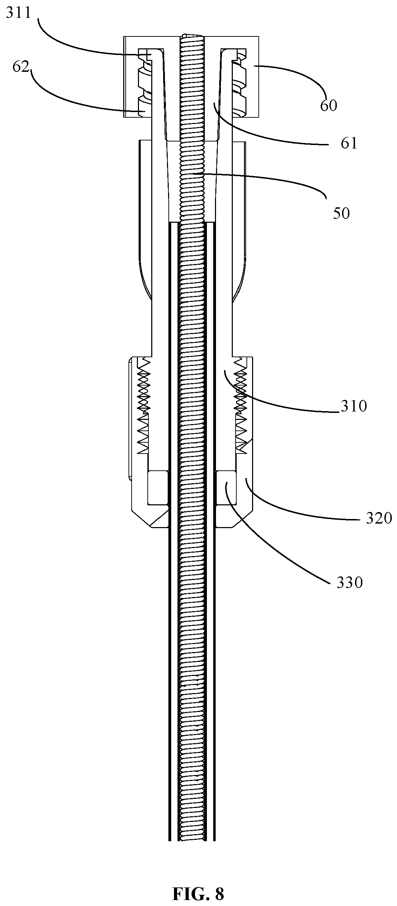

In the optional embodiments, as shown in FIGS. 5, 6, 7 and 8, the drainage tube further includes a first support pipe 50 and a connection end cap 60; the first support pipe 50 is disposed as passing through the interior of the drainage tube body 40 and the interior of the drainage portion 10, and the first support pipe 50 is configured to support the inner walls of the drainage portion 10 and the drainage tube body 40. The first support pipe provides evenly distributed support force, effectively enhances a structural strength of the entire drainage tube, and can effectively prevent the drainage tube from deforming or collapsing due to external pressure or bending during mounting and use. The first support pipe 50 can directly support the inner walls of the drainage portion 10 and the drainage tube body 40, and prevent the inner wall from wrinkling or collapsing when subjected to external press or internal negative pressure, the stability and smoothness of the inner wall are conducive to maintain the unobstructed flow of the drainage channel, reduce the risk of blockage, and improve the drainage efficiency. Additionally, as the first support pipe 50 provides additional support, the drainage tube body 40 and the drainage portion 10 can be made of softer and more biocompatible materials, thereby enhancing the comfort and safety of patients.

The connection of the connection end cap 60 with the proximal end of the first support pipe 50 realizes a stable connection and convenient disassembly and assembly between the first support pipe 50 and the drainage tube body 40. Specifically, when it is required to pull out the first support pipe 50 from the drainage tube 100, the user can drive the first support pipe 50 by means of the connection end cap 60 to pull out the first support pipe 50.

Furthermore, a central part of the connection end cap 60 protrudes outward to form an insertion portion 61. The insertion portion 61 can be inserted into the connector 300 to achieve a plug-in fixation between the connection end cap 60 and the connector 300, thereby facilitating the insertion mounting and withdrawal of the first support pipe 50.

The first support pipe 50 can be configured as a solid structure woven from multiple steel wires. A guidewire cannot pass through the first support pipe 50. The first support pipe 50 can also be configured as a hollow structure made by helically winding a single steel wire. When the first support pipe 50 is configured as a hollow structure, a hollow space is formed inside. The hollow space can be configured to accommodate the guidewire. The connection end cap 60 is provided with a through hole. The through hole is communicated with the hollow space inside the first support pipe 50, thus forming a communication channel from the outside of the connection end cap 60 to the inside of the first support pipe 50. The communication channel provides a smooth path for the passage of the guidewire, facilitating the placement of the drainage tube 100 into the body through the guidance of the guidewire during operation.

In addition, it should be noted that when the drainage tube 100 is implanted into the fistula tract by the surgical method adopting a guidewire, the first support pipe 50 is configured to be enclosable to form a hollow space for the guidewire to pass through. When the drainage tube 100 is implanted into the fistula tract by the surgical method adopting the combination of endoscopy and foreign body forceps, the first support pipe 50 can be configured to be enclosable to form a hollow space. The first support pipe 50 can also be configured as a solid structure woven from multiple steel wires.

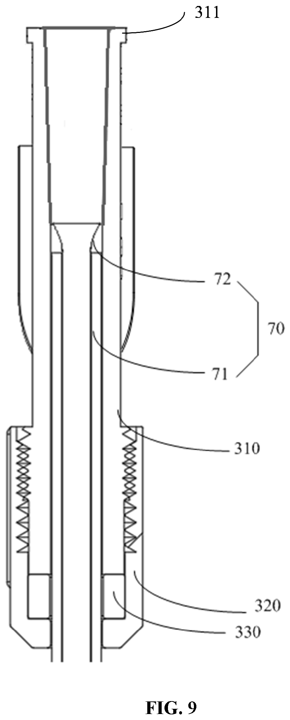

The connector 300 is in particular configured as a Ruhr connector, which includes a male connector 310, a female connector 320 and a sealing ring 330 arranged inside the female connector 320. The female connector 320 is closer to the distal end than the male connector 310. After the drainage tube 100 is placed into the body, the drainage tube 100 needs to be connected to the negative pressure device 200. The connection end cap 60 and the male connector 310 are loosened. Then, the first support pipe 50 is pulled out from the drainage tube 100. If the drainage tube is placed by means of a guidewire, the first support pipe 50 and the guidewire should be pulled out together. Thereafter, the connector 300 is detached from the drainage tube 100. Thus, the proximal end of the drainage tube 100 can be passed out through the nostril. Then, the connector 300 is assembled onto the drainage tube 100: first, the female connector 320 is sleeved onto the drainage tube 100; then, the male connector 310 is screwed. The male connector 310 is inserted into the female connector 320 with thread. The male connector 310 compresses the sealing ring 330. The sealing ring 330 is deformed under force and presses against the drainage tube 100, thereby fixedly assembling the connector 300 onto the drainage tube 100. However, due to the deformation of the sealing ring 330 under force, the sealing ring 330 will press against the drainage tube 100, causing the drainage tube 100 to deform. As a result, the channel at the deformed part of the drainage tube 100 becomes very small or even blocked, thereby affecting the subsequent drainage effect. Therefore, to solve this technical problem, as shown in FIG. 9, the drainage device further includes a second support pipe 70. The second support pipe 70 includes a straight barrel section 71 and an overlap section 72 connected with each other. The straight barrel section 71 is configured to be arranged inside the proximal end of the drainage tube 100. Before assembling the connector 300, the straight barrel section 71 is first placed inside the drainage tube 100. The size of the overlap section 72 is larger than the proximal end opening of the drainage tube 100, so that the overlap section 72 can abut against the drainage tube 100, preventing the second support pipe 70 from sliding into the drainage tube 100 and being difficult to remove. It should be noted that, in order to ensure that the straight barrel section 71 can withstand the pressing force of the sealing ring 330, the length of the straight barrel section 71 extending into the drainage tube 100 is greater than (beyond) the position of the sealing ring 330, ensuring that the straight barrel section 71 is present at the pressing position of the sealing ring 330. Thus, by providing the second support tube 70, the proximal end part of the drainage tube 100 is supported by the second support tube 70, so as to effectively prevent the channel at the deformation position of the drainage tube 100 from becoming very small or even blocked when assembling the connector 300.

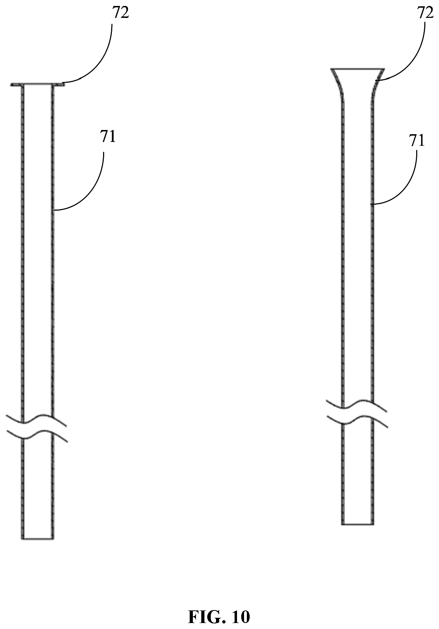

As shown in FIG. 10, the overlap section 72 can be configured in various shapes, such as in a trumpet shape or a right-angle shape. The specific shape structure of the overlap section 72 can be selected according to the actual situation.

In addition, to enhance the anti-deformation capacity of the second support pipe 70, the second support pipe 70 is made of a material having a certain rigidity. Preferably, the second support pipe 70 is configured as a metal material. A lug 311 is provided at the proximal end of the male connector 310. A slide groove 62 is provided on the inner wall of the connection end cap 60. The slide groove 62 is in a spiral shape. The lug 311 can slide in the slide groove 62, thus enabling the connection end cap 60 to be mounted on the male connector 310 by screwing.

In summary, the embodiments of the present application provide a drainage tube 100 and a drainage device 1000. The drainage tube 100 comprises a drainage portion 10, a porous loose structure 20 and a granulation barrier structure 30. The drainage portion 10 has a first drainage channel 11, and the drainage portion 10 is provided with a first drainage opening 12 in communication with the first drainage channel 11. The drainage portion 10 is covered with the porous loose structure 20. The porous loose structure 20 is covered with the granulation barrier structure 30. The granulation barrier structure 30 has a second drainage opening 31. The second drainage opening 31, the porous loose structure 20, the first drainage opening 12 and the first drainage channel 11 are in communication.

It is easy to understand that, the porous loose structure 20 serves to communicate the second drainage opening 31 with the first drainage opening 12. During negative pressure drainage therapy, liquids such as the wound exudate at the fistula can pass through the second drainage opening 31, the porous loose structure 20 and the first drainage opening 12, and enter the first drainage channel 11 of the drainage portion 10. Then, under the effect of negative pressure, the liquid is drawn out of the drainage tube 100. Moreover, as the porous loose structure 20 is covered with the granulation barrier structure 30, the granulation can be prevented from growing into the porous loose structure 20 to a certain extent. Thus, during the subsequent removal of the drainage tube 100, the pulling between the granulation tissue and the granulation barrier structure 30 as well as the porous loose structure 20 can be avoided, thereby reducing the bleeding of the granulation tissue. The therapeutic effect of negative pressure drainage has been improved.

The drainage device 1000 includes the drainage tube 100, which possesses all the functions and beneficial effects of the drainage tube 100.

The above description is merely a specific embodiment of the present application and is not intended to limit the present application. For those skilled in the art, the present application can be modified and changed in various ways. Any modification, equivalent substitution, improvement, etc. made within the spirit and principles of the present application shall be included within the scope of protection of the present application.

### INDUSTRIAL APPLICABILITY

In summary, the embodiments of the present application provide a drainage tube and drainage device. With the provision of a granulation barrier structure, not only the requirements of negative pressure adsorption can be met, but also the granulation tissue is prevented from growing into the porous loose structure, thereby reducing the bleeding of granulation tissue and improving the therapeutic effect of negative pressure drainage.

## Claims

1. A drainage tube, **characterized by** comprising:
a drainage portion (10), wherein the drainage portion (10) has a first drainage channel (11), and the drainage portion (10) is provided with a first drainage opening (12) in communication with the first drainage channel (11);
a porous loose structure (20), wherein the drainage portion (10) is covered with the porous loose structure (20); and
a granulation barrier structure (30), wherein the porous loose structure (20) is covered with the granulation barrier structure (30), and the granulation barrier structure (30) is provided with a second drainage opening (31),
wherein the second drainage opening (31), the porous loose structure (20), the first drainage opening (12) and the first drainage channel (11) are in communication.

2. The drainage tube according to claim 1, **characterized in that** a plurality of first drainage openings (12) are provided, and each of the plurality of first drainage openings (12) is in communication with the first drainage channel (11) and the porous loose structure (20), and wherein a plurality of the second drainage openings (31) are provided, and each of the plurality of second drainage openings (31) is communicated with the porous loose structure (20).

3. The drainage tube according to claim 2, **characterized in that** the plurality of first drainage openings (12) are arranged at intervals along an extension direction of the drainage portion (10); and/or
the plurality of second drainage openings (31) are arranged at intervals along an extension direction of the granulation barrier structure (30).

4. The drainage tube according to claim 2 or 3, **characterized in that** the second drainage opening (31) is in number which is greater than or equal to that of the first drainage opening (12), and a diameter of the second drainage opening (31) is smaller than that of the first drainage opening (12).

5. The drainage tube according to claim 1, **characterized in that** the drainage tube (100) further comprises a drainage tube body (40), wherein a distal end of the drainage tube body (40) is connected to a proximal end of the drainage portion (10), and the drainage tube body (40) has a second drainage channel (41) in communication with the first drainage channel (11).

6. The drainage tube according to claim 5, **characterized in that** a distal end of the drainage portion (10) is provided with a first opening (13) in communication with the first drainage channel (11), wherein a proximal end of the drainage tube body (40) is provided with a second opening (42) in communication with the second drainage channel (41).

7. The drainage tube according to claim 5 or 6, **characterized in that** a hardness of the proximal end of the drainage tube body (40) is greater than or equal to that of the drainage portion (10).

8. A drainage device, **characterized by** comprising the drainage tube (100) according to any one of claims 1 to 7 and a negative pressure device (200), and the negative pressure device (200) is connected to the proximal end of the drainage portion (10) and is in communication with the first drainage channel (11).

9. The drainage device according to claim 8, **characterized in that** the drainage device (1000) further comprises a connector (300), wherein the connector (300) is detachably arranged at the proximal end of the drainage portion (10), and the negative pressure device (200) is connected to a proximal end of the connector (300).

10. The drainage device according to claim 9, **characterized in that** the drainage device further comprises a first support pipe (50) and a connection end cap (60);
wherein the first support pipe (50) is disposed as passing through an interior of the drainage tube (100), and the first support pipe (50) is configured to support an inner wall of the drainage tube (100); and
wherein the connection end cap (60) is connected to a proximal end of the first support pipe (50), and a central protrusion of the connection end cap (60) forms an insertion portion that can be inserted into the connector (300).

11. The drainage device according to claim 10, **characterized in that** the first support pipe (50) is configured as a solid structure,
or, the first support pipe (50) is configured as a hollow structure to form a hollow space, and the connection end cap (60) has a through hole communicated with the hollow space to form a communication channel for a guidewire to pass through.

12. The drainage device according to any one of claims 8 to 11, **characterized in that** the drainage device (1000) further comprises a second support pipe (70);
wherein the second support pipe comprises a straight barrel section (71) and an overlap section (72) connected with each other;
wherein the straight barrel section (71) is configured to be arranged inside a proximal end of the drainage tube (100) to support the drainage tube (100); and
wherein the overlap section (72) is configured to abut against a proximal end opening of the drainage tube (100).
